# EUROPEAN PATENT SPECIFICATION

(11) **EP 2 758 021 B1**
(45) Date of publication and mention of the grant of the patent: **27.09.2017**
(21) Application number: 12715157.9
(22) Date of filing: 07.03.2012
(51) Int. Cl.: A61H 33/00, A61H 33/02, A61N 5/06

(54) **HYDROMASSAGE BATHTUB**
HYDROMASSAGEWANNE
BAIGNOIRE D'HYDROMASSAGE

(30) Priority: 19.09.2011 IT RM20110143
(43) Date of publication of application: 30.07.2014
(73) Proprietor: GRUPPO TRE S S.p.A., 01036 Nepi (VT) (IT)
(72) Inventor: SADLER, Marc, 01033 Civita Castellana (VT) (IT)
(74) Representative: Romano, Giuseppe
(86) International application number: PCT/IB2012/051056
(87) International publication number: WO 2013/041978

(56) References cited:
- EP-A1- 1 586 292
- US-A- 4 339 833
- US-A- 4 340 982
- US-A- 4 893 362

## Description

The present invention lies in the field of bathroom fittings and finds application in particular within the scope of the manufacturing of hydromassage bathtubs.

More specifically, the present description refers to a hydromassage bathtub, whose features make it particularly easy and pleasant to use, with a concomitant improvement of its functionality and therefore also of its effectiveness in terms of therapeutic treatment.

More and more often, in the last few years, it is fashionable to install hydromassage bathtubs; not merely in wellness and/or physiotherapy centers, but also in household bathroom premises and/or in hotels.

Accordingly, new designs have been sought ever more, both in terms of aesthetic appreciation and under a more typically technical and functional point of view.

In other words, the need particularly felt today is that of a product which may effectively combine cutting-edge technical/functional aspects with a particularly pleasing external appearance and an ease and practicality of use improved with respect to the past.

On the contrary, to date producers tend to add complexity to their bathtubs, on the one hand making them more effective and multi-function, yet on the other hand having to give up any expectation to obtain a product of pleasing appearance.

Suffice it to consider the presence of emission nozzles for emitting hydromassage jets. The number of nozzles tends to increase in order to improve effectiveness and provide greater flexibility of use, and therefore a greater effectiveness of the therapy.

However, nozzles have to find place on the internal walls of the bathtub; therefore, with an increase in their number, there increases the presence of projecting elements, or of elements anyhow interrupting the surface of the walls themselves.

Oft-times, this is accompanied by the presence of other elements, such as, e.g., lights, loudspeakers, or other accessories, besides of course components always present in the bathtubs, such as taps and fittings, drains, regulators of the level of fluid in the bathtub, etc.

US4340982A discloses an hydrotherapy bathtub having, along its perimeter, a series of holes from which water under pressure mixed with air should pass. Water under pressure is pumped in a conduct running outside the bathtub and having, in turn, holes facing in a second conduct where air is pumped.

US4893362A and US44339833A disclose hydromassage bathtubs having, along their perimeter, a series of nozzles for the hydromassage operation.

Hence, object of the present invention is to solve such problems, by means of a hydromassage bathtub, whose features are as defined in claim 1 and which allows to solve the problems left open by the known art.

Preferred features of the present invention are reported in the dependent claims.

The advantages, as well as the features and the modes of use of the present invention, will be made evident in the following detailed description related to possible embodiments thereof, given by way of example and not for limitative purposes. Reference will be made to the figures of the annexed drawings, wherein:
- Figure 1 is a perspective view of a first embodiment of a bathtub according to the present invention;
- Figure 2 is a partially sectional perspective view of the bathtub of Figure 1;
- Figure 3A is a partial sectional view of an edge of the bathtub of Figure 1;
- Figure 3B is a detail of Figure 3A;
- Figure 4A is a remote control that can be used for managing the functions of a bathtub according to the present invention;
- Figure 4B is a partial sectional view of Figure 4A;
- Figure 5A is a perspective view of a second embodiment of a bathtub according to the present invention;
- Figure 5B is a detail of Figure 5A; and
- Figure 6 is a partial sectional view of an edge of the bathtub of Figure 5A.

To describe the present invention, hereinafter reference will be made to the above-indicated figures.

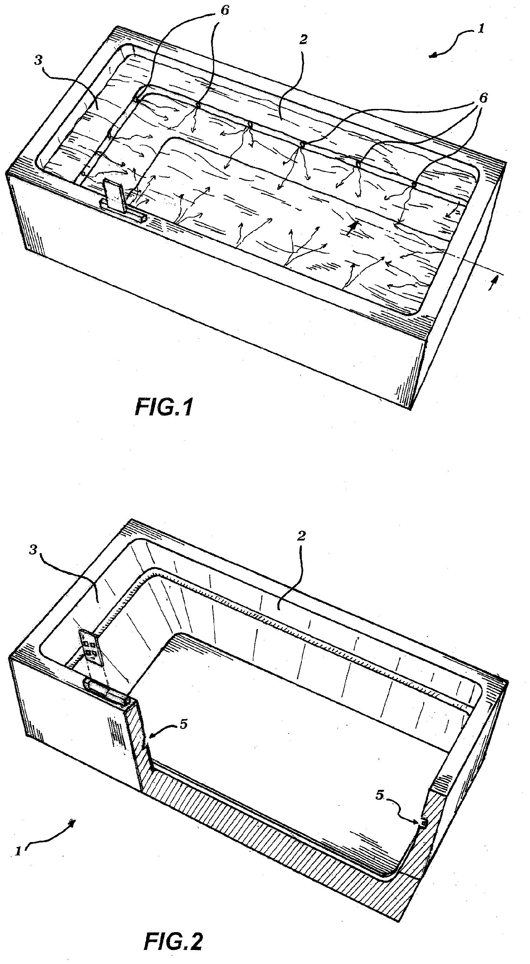

Referring initially to Figure 1, it shows a first embodiment of a bathtub 1 according to the present invention.

It is understood that the present invention, as it will be described hereinafter, may be applied for the manufacturing of bathtubs of any shape. Therefore, the shape of the bathtubs depicted herein should be construed as exclusively illustrative and by way of example.

The hydromassage bathtub 1 has one or more internal side walls 2, 3.

Along the bathtub perimeter, regardless of its shape, a recess 5 is provided, obtained at the internal side walls.

The side walls are therefore suitably shaped to cooperate with such recess 5 to make it suitable to carry out a housing function for a plurality of emission nozzles 6, for carrying out the hydromassage function.

Preferably, the perimetrical recess 5 is positioned substantially at one-half of the height of said internal side walls.

As it will be explained hereinafter, the perimetrical recess 5 may also constitute a seat for a plurality of lighting elements. Advantageously, the lighting elements 10, for instance a multi-color LED, etc., are arranged along the perimetrical recess 5, so as to light up the inside of said bathtub 1 for implementing a chromotherapy function.

As it will be explained hereinafter, the perimetrical recess 5 may also constitute a seat for a plurality of lighting elements. Advantageously, the lighting elements 10, e.g. a multi-color LED, etc., are arranged along the perimetrical recess 5, so as to light up the inside of said bathtub 1 for implementing a chromotherapy function.

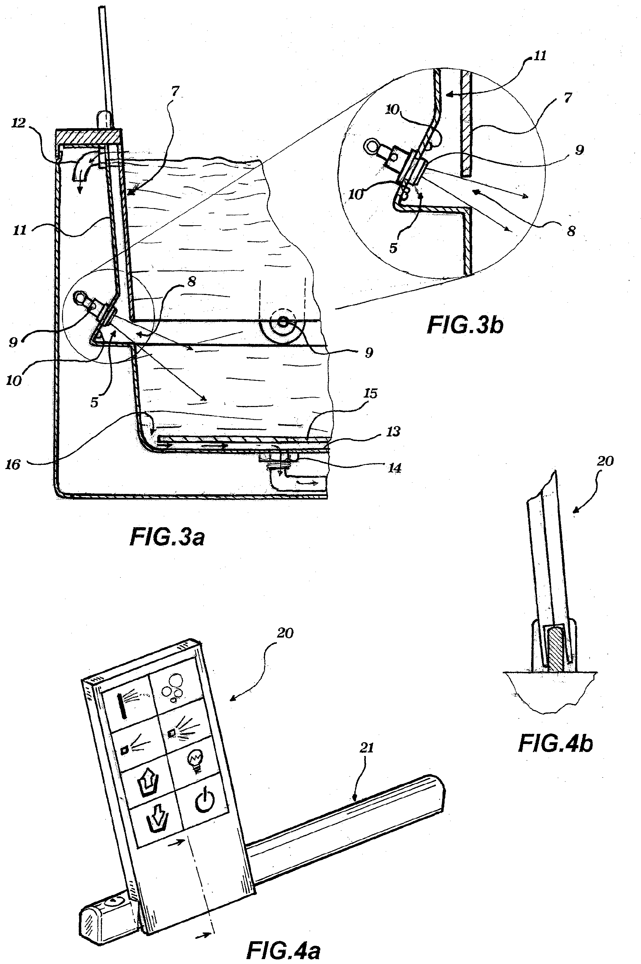

Next figures 3A and 3B show in greater detail the making of the recess 5 and the configuration of the side walls thereat.

In particular, the bathtub comprises one or more wall portions 7 set to at least partially cover the perimetrical recess 5. Thus, cooperation between the recess and the wall portions 7 defines a perimetrical slot 8 open to the inside of the bathtub 1.

Therefore, a plurality of emission nozzles 9 may advantageously be provided, said nozzles being housed within the recess 5. Preferably, the emission nozzles are of steerable type, so as to obtain the desired effect and the best hydromassage performances in terms of treatment.

The nozzles 9 may be distributed along the entire perimeter of the bathtub, within the perimetrical recess 5.

Advantageously, the wall portions 7 are at least partly spaced from the respective side walls, so as to define corresponding air spaces 11 internal to the walls themselves.

Of course, with a filled bathtub, the air space 11 will be flooded. Advantageously, the air space comprises a first discharge duct 12 for adjusting a maximum level of fluid in the bathtub 1, so as to carry out an "overflow" functionality for the bathtub 1.

Moreover, the bathtub 1 has a bottom 13 comprising a second discharge duct 14 for the emptying of the bathtub 1.

According to an alternative embodiment, the bathtub 1 further provides a false bottom element 15, overlapped to and spaced apart from the bottom 13.

The extension and the arrangement of the false bottom 15 is such as to define a perimetrical discharge opening 16 extending along the perimeter of the false bottom element 15 itself.

Advantageously, the bathtub 1 may comprise means for emitting gas from the perimetrical discharge opening 16 to the inside of the bathtub 1. E.g., air, which could be aromatized with different essences and will serve to amplify the beneficial effect of the hydromassage, could be inlet into the bathtub.

According to further embodiments, the presence of an audio diffusion system may advantageously be provided, e.g. for file reproduction, e.g. in MP3 format, or for radio listening.

Preferably, each bathtub function could be operated by electronically controlled electric or electromechanical actuators.

E.g., the taps and fittings for bathtub filling, as well as the drain ducts, may be opened and/or closed by means of electronically controlled electrovalves.

To this end, an electronic management system may advantageously be provided for the opening/closing of filling and draining ducts and/or the adjusting of the temperature of the fluid filling the bathtub and/or the opening/closing of the emission nozzles and/or the on/off switching of the lighting elements.

Such electronic system could therefore be operated by the user, for the activating/deactivating of each functionality of the bathtub. This may occur, e.g., by means of a remote control 20, e.g. like that illustrated in Figures 4A and 4B.

The remote control 20 may be housed on a support 21 which, preferably, is set on an edge of the bathtub 1, well within reach of a user's hand.

Therefore, on the remote control 20 it is provided a panel of keys bearing icons descriptive of the implemented function.

We do not deem it necessary to delve into the details of the implementation of the remote control and/or of the management system, the various functionalities being variable from design to design or customizable according to users' needs.

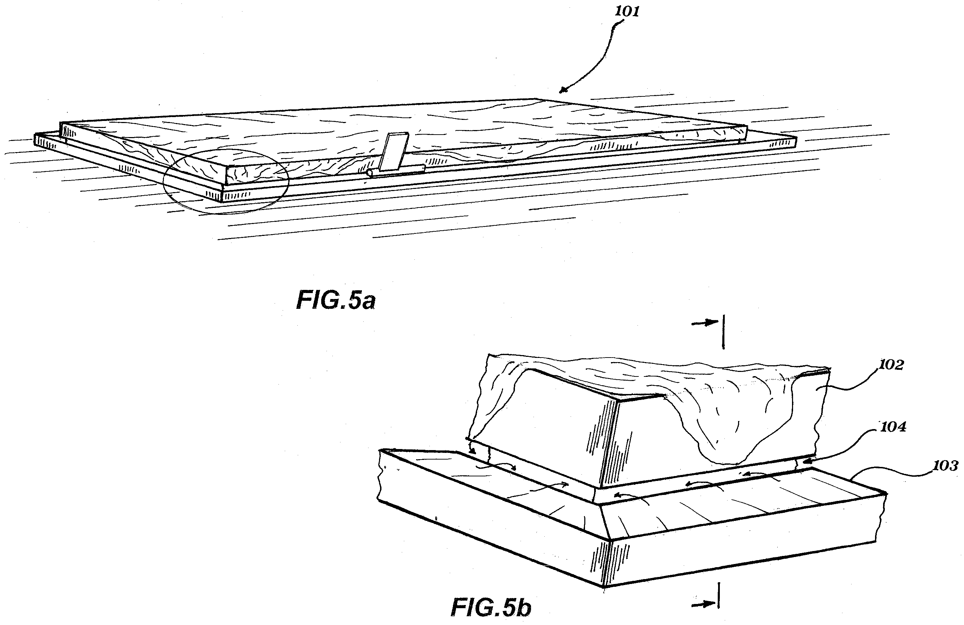

Figures 5A and 5B refer to a further embodiment of a bathtub 101 according to the present invention.

In particular, the bathtub 101 may have, taken individually or in conjunction, all of the features described hereto in connection to the preceding embodiments.

In addition, the bathtub 101 comprises a system for adjusting a maximum level of fluid into the bathtub 101 of perimeter-overflow type.

Therefore, upon filling the bathtub 101, water will overflow the edge 102, as schematically illustrated in Figure 5B, to end into a perimetrical discharge duct 104, obtained between the edge 102 and a second edge 103. Of course, the shape and slopes of the edges 102 and 103 will be such as to guarantee water outflow to the drain 104.

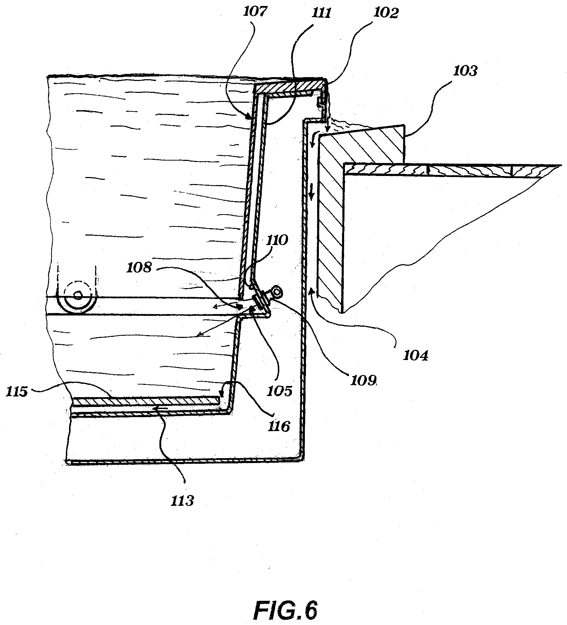

Next Figure 6 is a partial sectional view of an edge of the bathtub 101 of Figure 5A.

It will be immediately appreciated that, as mentioned hereto, the bathtub 101 envisages the presence of features similar or identical to what has been described hereto.

In fact, a perimetrical recess 105 is provided, at least partially faced by a wall portion 107, to form a slot 108 open to the inside of the bathtub 101.

Inside the recess 105 a plurality of emission nozzles 109, preferably of steerable type, and a plurality of lighting elements 110, preferably multi-color LEDs, may be housed.

On the bottom of the bathtub 101 a false bottom element 115 may be provided, spaced from the bottom of the bathtub 101, defining a perimetrical drain opening 116.

Of course, it is understood that also this embodiment of the bathtub may be completely managed, in all of its functions, by an electronic management system, preferably by means of a remote control, exactly as described in the foregoing.

The present invention has hereto been described with reference to preferred embodiments thereof. It is understood that other embodiments might exist, all falling within the concept of the same invention, as defined by the protective scope of the claims hereinafter.

## Claims

1. A hydromassage bathtub (1, 101) comprising:
one or more internal side walls;
a perimetrical recess (5, 105) extending substantially along all of the perimeter of the bathtub (1, 101) at said internal side walls, said perimetrical recess (5, 105) being apt to house a plurality of angled downward emission nozzles (9, 109) arranged along said perimetrical recess (5, 105) for carrying out the hydromassage function;
at least one wall portion (7, 107) set to at least partially cover said perimetrical recess (5, 105), so as to define a perimetrical slot (8, 108) open towards the inside of said bathtub (1, 101);
a bottom (13, 113) comprising a drain duct (14, 114) for draining liquid from the bathtub (1, 101);
a false bottom element (15, 115), overlapped to and spaced apart from said bottom (13, 113), the extension and the arrangement of said false bottom (15, 115) being such as to define a perimetrical drain opening (16, 116) extending along the perimeter of the false bottom element (15, 115) itself;
means for emitting gas from said perimetrical drain opening (16, 116) to the inside of said bathtub (1, 101),
wherein said at least one wall portion (7, 107) is spaced apart from a respective side wall to define a corresponding air space (11, 111) internal to the respective side wall.

2. The bathtub according to claim 1, wherein said perimetrical recess (5, 105) is positioned substantially at one-half of the height of said internal side walls.

3. The bathtub according to claim 1 or 2, wherein said perimetrical recess (5, 105) is also a seat for a plurality of lighting elements (10, 110).

4. The bathtub (1) according to anyone of the preceding claims, wherein said air space (11) comprises a first discharge duct (12) for adjusting a maximum level of fluid in the bathtub (1).

5. The bathtub (101) according to one of the claims 1 to 4, further comprising a perimeter-overflow system (102, 103, 104) for adjusting a maximum level of liquid in the bathtub (101).

6. The bathtub according to claim 5, wherein said emission nozzles (9, 109) are of steerable type, so as to be able to steer the jet thereof to the inside of said bathtub (1, 101).

7. The bathtub according to one of the claims 1 to 6, further comprising a plurality of lighting elements (10, 110) arranged along said perimetrical recess (5, 105), so as to light up the inside of said bathtub (1, 101).

8. The bathtub according to one of the claims 1 to 7, further comprising an audio diffusion system.

9. The bathtub according to one of the claims 1 to 8, further comprising an electronic management system, for the opening/closing of filling and draining ducts and/or the adjusting of the temperature of the liquid filling the bathtub and/or the opening/closing of the emission nozzles and/or the on/off switching of lighting elements.

## Patentansprüche

1. Hydromassagebadewanne (1, 101), umfassend:
eine oder mehrere innere Seitenwände;
eine Umfangsausnehmung (5,105), die sich im Wesentlichen entlang aller Umfänge der Badewanne (1, 101) an den inneren Seitenwänden erstreckt, wobei die Umfangsausnehmung (5, 105) geeignet ist, eine Vielzahl von nach unten abgewinkelten Ausströmdüsen (9, 109) aufzunehmen, die entlang der Umfangsausnehmung (5, 105) angeordnet sind, um die Hydromassagefunktion auszuführen;
wobei wenigstens ein Wandabschnitt (7, 107) eingerichtet ist, um wenigstens teilweise die Umfangsausnehmung (5, 105) abzudecken, um so einen Umfangsschlitz (8, 108) zu definieren, der offen zu der Innenseite der Badewanne (1, 101) ist;
wobei ein Boden (13, 113) eine Abflussführung (14, 114) umfasst, um Flüssigkeit aus der Badewanne (1, 101) abfließen zu lassen;
ein Falschbodenelement (15, 115), das den Boden (13, 113) überlappt, und davon einen Abstand aufweist, wobei die Erweiterung und die Anordnung des Falschbodens (15, 115) so ist, dass er eine Umfangsabflussöffnung (16, 116) definiert, die sich entlang dem Umfang des Falschbodenelementes (15, 115) selber erstreckt;
Mittel zum Ausgeben von Gas von der Umfangsabflussöffnung (16, 116) zu der Innenseite der Badewanne (1, 101),
wobei der wenigstens eine Wandabschnitt (7, 107) eine Abstand von einer jeweiligen Seitenwand aufweist, um einen entsprechenden Luftraum (11, 111) im Inneren der jeweiligen Seitenwand zu definieren.

2. Badewanne nach Anspruch 1, wobei die Umfangsausnehmung (5, 105) im Wesentlichen bei einer Hälfte der Höhe der inneren Seitenwände angeordnet ist.

3. Badewanne nach Anspruch 1 oder 2, wobei die Umfangsausnehmung (5, 105) auch ein Sitz für eine Vielzahl von Beleuchtungselementen (10, 110) ist.

4. Badewanne (1) nach einem der vorhergehenden Ansprüche, wobei der Luftraum (11) eine erste Auslaufführung (12) zum Einstellen eines maximalen Flüssigkeitsniveaus in der Badewanne (1) umfasst.

5. Badewanne (101) nach einem der Ansprüche 1 bis 4, die weiterhin ein Umfangsüberlaufsystem (102, 103, 104) zum Einstellen eines maximalen Flüssigkeitsniveaus in der Badewanne (101) umfasst.

6. Badewanne nach Anspruch 5, wobei die Ausströmdüsen (9, 109) steuerbar sind, um geeignet zu sein, deren Strahl zur Innenseite der Badewanne (1, 101) zu steuern.

7. Badewanne nach einem der Ansprüche 1 bis 6, die weiterhin eine Vielzahl von Beleuchtungselementen (10, 110) umfasst, welche entlang der Umfangsausnehmung (5, 105) angeordnet sind, um so die Innenseite der Badewanne (1, 101) zu beleuchten.

8. Badewanne nach einem der Ansprüche 1 bis 7, die weiterhin ein Audiodiffusionssystem umfasst.

9. Badewanne nach einem der Ansprüche 1 bis 8, die weiterhin ein elektronisches Managementsystem umfasst, zum Öffnen/Schließen von Füll- und Abflussführungen und/oder zum Einstellen der Temperatur der Flüssigkeit, welche die Badewanne füllt, und/oder zum Öffnen/Schließen der Ausströmdüsen und/oder zum Ein-/Ausschalten der Beleuchtungselemente.

## Revendications

1. Baignoire d'hydromassage (1, 101) comprenant :
une ou plusieurs parois latérales internes ;
un évidement périmétral (5, 105) s'étendant sensiblement le long de tout le périmètre de la baignoire (1, 101) au niveau desdites parois latérales internes, ledit évidement périmétral (5, 105) étant apte à loger une pluralité de buses d'émission inclinées vers le bas (9, 109) agencées le long dudit évidement périmétral (5, 105) pour réaliser la fonction d'hydromassage ;
au moins une partie de paroi (7, 107) placée pour recouvrir au moins partiellement ledit évidement périmétral (5, 105), afin de définir une fente périmétrale (8, 108) ouverte vers l'intérieur de ladite baignoire (1, 101) ;
un fond (13, 113) comprenant un conduit de vidange (14, 114) pour vidanger le liquide de la baignoire (1, 101) ;
un élément de double fond (15, 115), recouvert et espacé dudit fond (13, 113), l'extension et l'agencement dudit double fond (15, 115) étant tels qu'ils définissent une ouverture de vidange périmétrale (16, 116) s'étendant le long du périmètre de l'élément de double fond (15, 115) lui-même ;
des moyens pour émettre du gaz par ladite ouverture de vidange périmétrale (16, 116) à l'intérieur de ladite baignoire (1, 101),
dans laquelle ladite au moins une partie de paroi (7, 107) est espacée d'une paroi latérale respective pour définir un espace d'air (11, 111) correspondant à l'intérieur de la paroi latérale respective.

2. Baignoire selon la revendication 1, dans laquelle ledit évidement périmétral (5, 105) est positionné sensiblement à la moitié de la hauteur desdites parois latérales internes.

3. Baignoire selon la revendication 1 ou 2, dans laquelle ledit évidement périmétral (5, 105) est également un siège pour une pluralité d'éléments d'éclairage (10, 110).

4. Baignoire (1) selon l'une quelconque des revendications précédentes, dans laquelle ledit espace d'air (11) comprend un premier conduit de décharge (12) pour ajuster un niveau maximum de fluide dans la baignoire (1).

5. Baignoire (101) selon l'une quelconque des revendications 1 à 4, comprenant en outre un système de trop-plein périmétral (102, 103, 104) pour ajuster un niveau maximum de liquide dans la baignoire (101).

6. Baignoire selon la revendication 5, dans laquelle lesdites buses d'émission (9, 109) sont de type orientable, afin de pouvoir orienter leur jet vers l'intérieur de ladite baignoire (1, 101).

7. Baignoire selon l'une des revendications 1 à 6, comprenant en outre une pluralité d'éléments d'éclairage (10, 110) agencés le long dudit évidement périmétral (5, 105), afin d'éclairer l'intérieur de ladite baignoire (1, 101).

8. Baignoire selon l'une des revendications 1 à 7, comprenant en outre un système de diffusion audio.

9. Baignoire selon l'une des revendications 1 à 8, comprenant en outre un système de gestion électronique, pour ouvrir/fermer les conduits de remplissage et de vidange et/ou l'ajustement de la température du liquide de remplissage de la baignoire et/ou l'ouverture/fermeture des buses d'émission et/ou la mise en marche/l'arrêt des éléments d'éclairage.
